# FASCICULE DE BREVET EUROPEEN

(11) **EP 1 282 384 B1**
(45) Date de publication et mention de la délivrance du brevet: **29.12.2004**
(21) Numéro de dépôt: 01936558.4
(22) Date de dépôt: 17.05.2001
(51) Int. Cl.: A61F 2/16, A61B 17/28

(54) **INJECTEUR POUR IMPLANT SOUPLE**
WERKZEUG ZUM IMPLANTIEREN EINES FLEXIBLEN IMPLANTATS
INJECTOR FOR FLEXIBLE IMPLANT

(30) Priorité: 19.05.2000 FR 0006417
(43) Date de publication de la demande: 12.02.2003
(73) Titulaire: CORNEAL INDUSTRIE, 74370 Pringy (FR)
(72) Inventeur: JEANNIN, Lionel, F-74330 Choisy (FR); BOS, Gilles, F-74330 La Balme de Sillingy (FR)
(74) Mandataire: Dronne, Guy
(86) Numéro de dépôt international: PCT/FR2001/001510
(87) Numéro de publication internationale: WO 2001/087186

(56) Documents cités:
- EP-A- 0 340 698
- EP-A- 0 363 213
- WO-A-95/13022
- WO-A-95/24863
- US-A- 4 165 746
- US-A- 4 976 716
- US-A- 5 052 402
- US-A- 5 454 818

## Description

La présente invention a pour objet un module de pliage d'implant souple et un injecteur d'implant souple utilisant le module de pliage.

Les implants souples qui sont réalisés le plus souvent en pHEMA ou en matériau à base de silicone présentent l'intérêt essentiel que, la partie optique de ces implants étant réalisée avec ce matériau souple, il est possible de plier l'implant et en particulier sa partie optique afin d'en réduire les dimensions transversales et de permettre ainsi l'introduction de l'implant dans l'oeil à travers une incision réalisée dans la cornée, cette incision ayant une dimension réduite typiquement de l'ordre de 3 mm.

Ces implants souples sont d'autant plus intéressants que les nouvelles techniques d'ablation du cristallin ne nécessitent plus que la réalisation d'une incision dans la cornée de dimension réduite de l'ordre de 3 mm lorsque l'on utilise la technique de phacoémulsifiation.

Cependant, le pliage de l'implant qui est constitué par sa partie optique sensiblement circulaire et par sa partie haptique, présente certaines difficultés notamment en raison des faibles dimensions de l'implant intraoculaire dont le diamètre de la partie optique est typiquement au plus égal à 6 mm et en raison de la nature du matériau constituant cet implant.

Pour permettre le pliage ou enroulement de l'implant avant l'insertion de l'implant dans l'oeil, on a déjà proposé différents types d'injecteurs d'implant souple incorporant un ensemble de pliage de cet implant.

Par pliage de l'implant, il faut entendre toutes les opérations mécaniques qui permettent de modifier élastiquement la forme de l'implant et notamment la forme de sa partie optique afin de réduire l'encombrement transversal de cet implant, c'est-à-dire dans la direction orthogonale à un diamètre de la partie optique contenant les éléments haptiques de l'implant. Il pourra s'agir d'un pliage simple autour d'un diamètre de la partie optique, d'un pliage plus complexe autour d'axes parallèles à ce diamètre ou encore d'un enroulement autour d'un axe parallèle à ce diamètre.

On comprend en effet qu'une fois l'implant plié, il suffit de placer une canule dans l'incision de l'oeil, cette canule prolongeant l'ensemble de pliage et de pousser cet implant via la canule sous forme pliée à l'intérieur de l'oeil.

Dans le document WO 95113022, on a déjà décrit un injecteur d'implant souple comportant un ensemble de pliage qui est constitué d'une part par une chambre de pliage comprenant une paroi incurvée de pliage et deux parois parallèles et d'autre part par un poussoir agissant sur un bord proximal de la partie optique de l'implant selon une direction orthogonale à la paroi de pliage, le poussoir, par son déplacement progressif, provoquant le pliage ou l'enroulement de l'implant.

Cependant, il apparaît que ce type d'ensemble de pliage ou d'enroulement de l'implant est mal adapté surtout lorsque cet implant comporte certains types de parties haptiques. On comprend en effet qu'il est nécessaire de plier ou enrouler la partie haptique, qui est le plus souvent constituée par deux éléments distincts, dans le même sens que la partie optique. Or, la partie haptique présente des dimensions réduites par rapport à celle de la partie optique et donc des propriétés de résistances mécaniques réduites rendant ce pliage de la partie haptique très délicat.

En outre, la mise en place de l'implant dans la chambre de pliage des modules de pliage connus est malaisée et le positionnement correct des parties haptiques est aléatoire.

Un objet de la présente invention est de fournir un module de pliage d'implant souple et un injecteur d'implant souple utilisant ce module de pliage qui permettent d'assurer dans des conditions améliorées le pliage ou l'enroulement non seulement de la partie optique mais également celui de la partie haptique de l'implant intraoculaire.

Selon l'invention, pour atteindre ce but, le module de pliage d'implant souple comprend :
deux mors mobiles l'un par rapport à l'autre, chaque mors comprenant une paroi de pliage constituée par une portion de surface cylindrique et une paroi plane solidaire de la paroi de pliage, les deux parois planes étant disposées dans des plans parallèles ;
un axe de rotation orthogonal aux parois planes pour contrôler le mouvement relatif de rotation d'un mors par rapport à l'autre ;
l'un des mors pouvant occuper par rapport à l'autre une première position dans laquelle les parois planes sont décalées l'une par rapport à l'autre, par quoi l'implant peut être mis en place sur la paroi plane inférieure ;
une deuxième position dans laquelle les parois planes sont en regard l'une de l'autre, par quoi les parois de pliage et les parois planes définissent un volume de confinement de l'implant; et
une troisième position dans laquelle les parois de pliage sont raccordées entre elles pour définir un volume sensiblement cylindrique formant une chambre de pliage permettant d'obtenir le pliage final de l'implant.

Grâce au mouvement relatif de rotation des deux mors autour de l'axe de pivotement, les parois de pliage des mors n'entrent pas en contact simultanément avec les deux éléments de la partie haptique de l'implant et avec sa partie optique. Comme on l'expliquera plus en détails ultérieurement, cette action décalée dans le temps sur les différentes parties de l'implant permet d'obtenir un pliage convenable de l'ensemble de l'implant quelle que soit la forme des éléments constituant la partie haptique.

En outre, la mise en place de l'implant dans le module se fait aisément lorsque les deux mors sont dans leur première position relative.

De préférence, le premier desdits mors comporte, en outre, deux parois latérales parallèles audit axe de rotation et solidaires des bords de la paroi plane dudit mors, par quoi lorsque les deux mors sont dans leur 2^{ème} position relative, les parois planes, les parois de pliage et les parois latérales des deux mors définissent un espace "fermé" de stockage dans lequel l'implant peut être maintenu dans un état non plié.

Grâce à cette disposition, l'implant peut être initialement placé dans la chambre de stockage, le module servant en même temps d'élément de confinement de l'implant. Le chirurgien n'a plus qu'à enlever l'emballage du module déjà équipé de son implant et à amener les deux mors dans leur troisième position relative pour pouvoir procéder à l'injection de l'implant dans l'oeil du patient.

L'invention concerne également un injecteur d'implants souples qui se caractérise en ce qu'il comprend :
- un module de pliage,
- un corps d'injecteur comprenant un logement pour recevoir ledit module de pliage, une canule d'injection pour prolonger ledit passage d'injection lorsque ledit module de pliage est en place dans ledit logement et un conduit axial de guidage pour prolonger ledit passage de guidage lorsque ledit module de pliage est en place dans ledit logement ; et
- un piston dont la tige est montée coulissante dans ledit conduit axial de guidage, par quoi l'extrémité de la tige dudit piston peut coopérer avec une extrémité de l'organe de poussage dudit module de pliage.

Selon un premier mode de réalisation de l'injecteur, le module de pliage est amovible par rapport au corps d'injecteur de telle manière que, après une utilisation de l'injecteur, il suffit de changer le module de pliage dans lequel l'implant a été initialement stocké. Dans un deuxième mode de mise en oeuvre, le module de pliage fait partie intégrante de l'injecteur et l'ensemble de l'injecteur doit être changé après chaque utilisation.

D'autres caractéristiques et avantages de l'invention apparaîtront mieux à la lecture de la description qui suit de plusieurs modes de réalisation de l'invention donnés à titre d'exemple non limitatifs.

La description se réfère aux figures annexées sur lesquelles :
- la figure 1 est une vue en coupe partielle d'un module de pliage en position ouverte ;
- la figure 2a est une vue en coupe longitudinale du module en position d'ouverture intermédiaire ;
- la figure 2b est une vue en coupe selon la ligne B-B de la figure 2a ;
- La figure 3a est une vue en coupe longitudinale du module en position finale de pliage;
- la figure 3b est une vue en coupe selon la ligne B-B de la figure 3b ;
- la figure 4 est une vue de dessus d'un injecteur d'implant selon l'invention ;
- La figure 5 est une vue en coupe longitudinale de l'injecteur de la figure 4 ;
- la figure 6 est une vue de côté de l'injecteur d'implant de la figure 4 ; et
- les figures 7a et 7b sont des schémas illustrant le pliage de l'implant dans le cas d'un injecteur standard et dans le cas d'un injecteur conforme à l'invention.

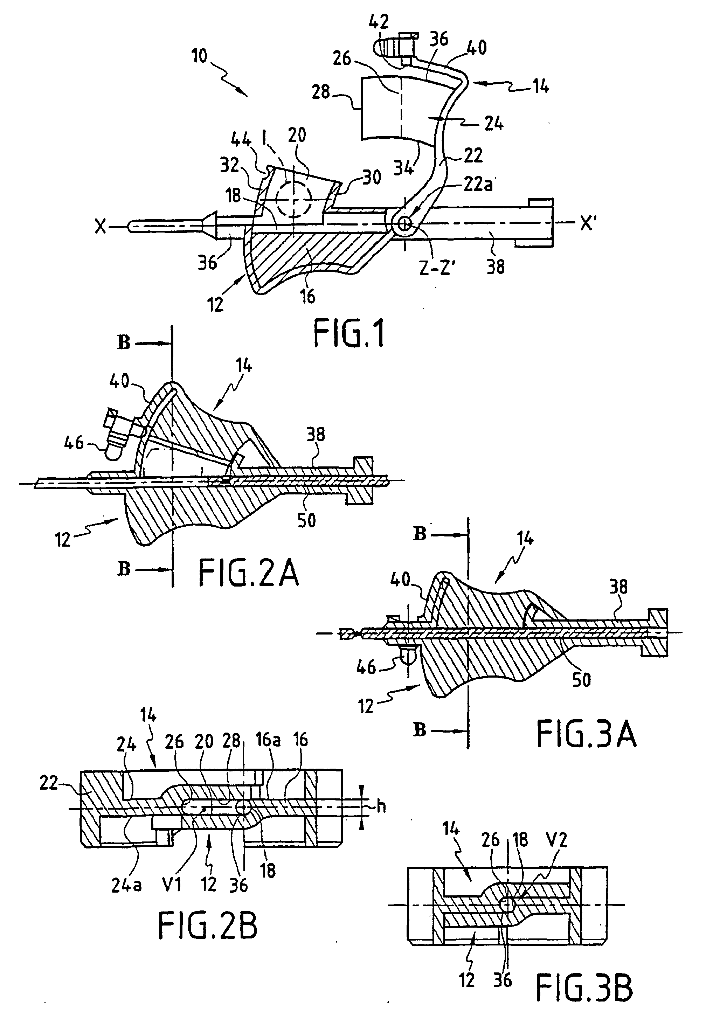

En se référant tout d'abord aux figures 1 à 3, on va décrire un mode préféré de réalisation du module de pliage d'implant souple. Le module 10 est essentiellement constitué par un premier mors 12 et par un deuxième mors 14 qui sont mobiles en rotation autour d'un axe. Le premier mors 12 comporte une base 16 qui définit une face de pliage 18 de forme hémi-cylindrique et d'axe X-X' ainsi qu'une extension définissant une face plane 20 reliée tangentiellement à la face de pliage 18. L'embase 16 sert à relier l'axe de rotation Z-Z' qui est orthogonal à l'axe longitudinal X-X' à la face de pliage 18 et à la face plane 20. Le deuxième mors 14 comporte également une embase 22 en forme de bras dont l'extrémité 22a est montée pivotante autour de l'axe Z-Z' et une extension 24 définissant une face de pliage 26 de forme sensiblement semi-cylindrique et une face plane 28. Ces différentes parties sont mieux visibles sur la figure 2b. Le premier mors 12 comporte également deux parois laterales 30 et 32 disposées de part et d'autre de la face plane 20. Ces parois laterales 30 et 32 ont la forme d'arcs de cercle centré sur l'axe Z-Z'. De la même manière, l'embase 24 et l'extension 26, 28 du deuxième mors 14 ont des bords 34 et 36 en forme d'arcs de cercle. Le premier mors 12 comporte également un premier passage d'injection 36 disposé selon l'axe X-X' et qui permet la sortie de l'implant une fois qu'il a été plié et un deuxième passage de guidage 38 qui permet le coulissement d'un organe de poussage pour permettre l'introduction de l'implant plié dans le passage d'injection 36.

Comme le montre mieux la figure 2b, les faces planes 20 et 28 des deux mors sont séparés par une distance h qui est supérieure à l'épaisseur de l'implant à plier et qui correspond au diamètre externe de l'implant après son pliage. Cette distance h correspond au diamètre interne du passage d'injection 36. De préférence, h est compris entre 1 et 3 mm. Comme le montre également la figure 2b, la face 24a de l'embase 24 peut glisser sur la face plane 20 de l'autre mors, il en va de même de la face 16a de l'embase 16 et de la face plane 28 de l'autre mors.

En outre, l'axe de rotation des mors Z-Z' coupe de préférence l'axe X-X' du mors 12.

Sur la figure 1, le deuxième mors 14 est représenté en position totalement écartée du premier mors 12. Dans cette position, il est possible de mettre aisément en place un implant I reposant sur la face plane 20 entre les parois latérales 30 et 32.

Le mors 14 peut être amené dans une deuxième position représentée sur la figure 2a dans laquelle le mors 14 est partiellement engagé avec le mors 12. Plus précisément, le bras 22 du mors 14 comporte une extension 40 munie d'un ergot 42 qui peut coopérer avec une encoche 44 prévue dans la face externe de la paroi latérale 32 du mors 12. On peut ainsi immobiliser en rotation le mors 12 par rapport au mors 14 dans une position telle que le volume V1 limité par les faces de pliage 26 et 18 et par les faces planes 20 et 28 et les parois latérales contiennent l'implant I sans que celui-ci soit plié. Cette position des deux mors constitue une position de confinement de l'implant qui permet éventuellement son stockage pendant une période convenable.

De plus, il est important de noter que, dans cette phase et dans la phase suivante (figures 3a et 3b), la face plane d'un mors est raccordée sensiblement tangentiellement à la face plane de l'autre mors. On évite ainsi tout point singulier lors du rapprochement mutuel des deux mors.

Pour obtenir le pliage de l'implant au sens où on l'a défini précédemment, on amène le mors 14 par rapport au mors 12 dans une troisième position dans laquelle les deux faces de pliage semi-cylindrique sont adjacentes pour définir un volume interne V2 de forme sensiblement cylindrique. Lors du passage de la position représentée sur la figure 2b à la position représentée sur la figure 3b, on comprend que le rapprochement des faces de pliage des deux mors provoque le pliage de l'implant, celui-ci étant maintenu entre les faces planes 20 et 28. Selon la nature et les dimensions de l'implant, ce pliage pourra être un pliage sensiblement selon un diamètre de la partie optique de l'implant ou un pliage plus complexe ou encore un enroulement autour d'un axe parallèle à l'axe X-X'. L'extrémité de l'extension 40 peut comporter un système de clipsage 46 coopérant avec la face externe du canal d'injection 36 pour maintenir le mors 14 dans sa position représentée sur la figure 3a, c'est-à-dire dans la position d'injection.

Le module de pliage représenté sur les figures 1 à 3 est destiné à être mis en place dans un corps d'injecteur pour permettre l'injection effective de l'implant. Dans ce cas, le canal de guidage 38 est équipé d'un poussoir 50 permettant d'introduire l'implant plié dans le canal de guidage 36 sous l'action d'un piston dont est équipé le corps de l'injecteur.

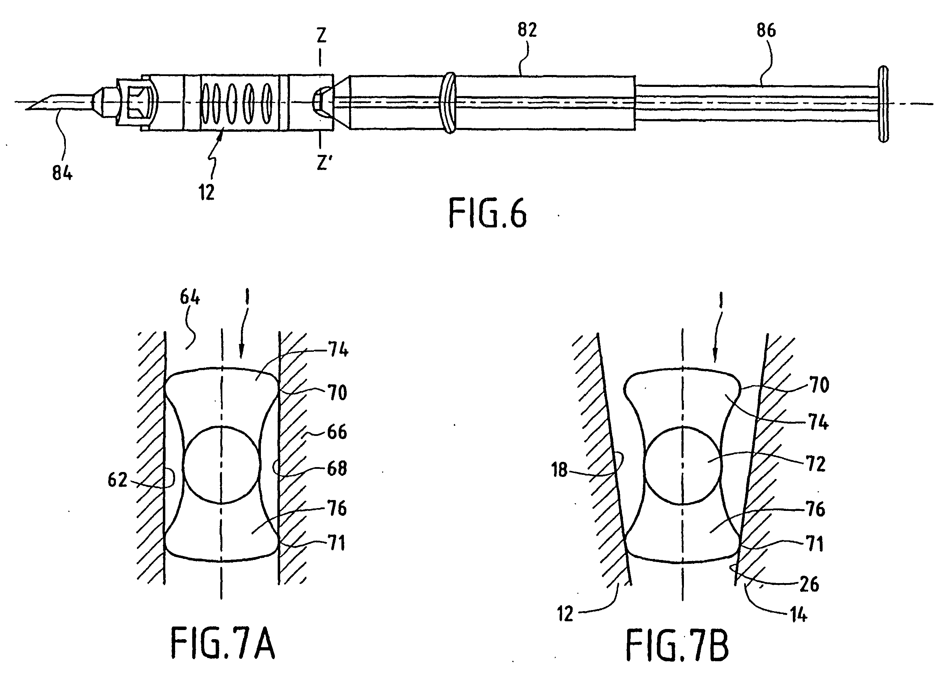

Sur la figure 7a, on a représenté le pliage d'un implant I par un module de pliage de type connu dans lequel on trouve une chambre de pliage 60 définissant une paroi de pliage de forme hémi-cylindrique 62 et deux parois parallèles dont seule la paroi 64 est visible. Le pliage est obtenu par le déplacement en translation rectiligne selon une direction orthogonale à la paroi de pliage 62 d'un poussoir 66 dont la face active 68 est également hémi-cylindrique. Lors du déplacement en translation rectiligne du poussoir 66, sa face de pliage 68 agit simultanément sur le bord proximal 70 et 71 de l'implant I, c'est-à-dire de ses parties haptiques 74 et 76. Ces bords proximaux 70 et 71 sont mécaniquement indépendants l'un de l'autre. Lorsque la face active 68 du poussoir 66 entre en contact avec un bord proximal, le pliage ou l'enroulement de la partie correspondante de l'implant peut se faire aléatoirement dans un sens ou dans l'autre. Comme les deux bords proximaux 70 et 71 sont mécaniquement indépendants, le risque consiste dans le fait que le sens d'initialisation du pliage ou de l'enroulement soit différent pour les deux parties haptiques 74 et 76. Ces sens de pliage différents pour les deux parties haptiques risquent d'altérer l'implant lorsque le pliage de l'implant se poursuit par rapprochement du poussoir 66 vers la face de pliage.

Dans le cas de la figure 7b qui correspond à l'invention, les mors 12 et 14 ont un mouvement relatif de rotation. En conséquence, lors du rapprochement de ces deux mors, les parois semi-cylindriques de pliage 18 et 26 entrent en contact d'abord avec une des parties haptiques 76 puis avec la partie optique 72 et enfin avec la partie haptique 74 du moins dans l'exemple considéré. Le fait que les parois de pliage entrent successivement en contact avec ces différentes parties de l'implant permet d'obtenir un sens de pliage initial pour une des parties de l'implant, sens de pliage qui se transmet lorsque les faces de pliage agissent sur les autres parties de cet implant, ce qui assure un pliage uniforme pour l'ensemble de l'implant.

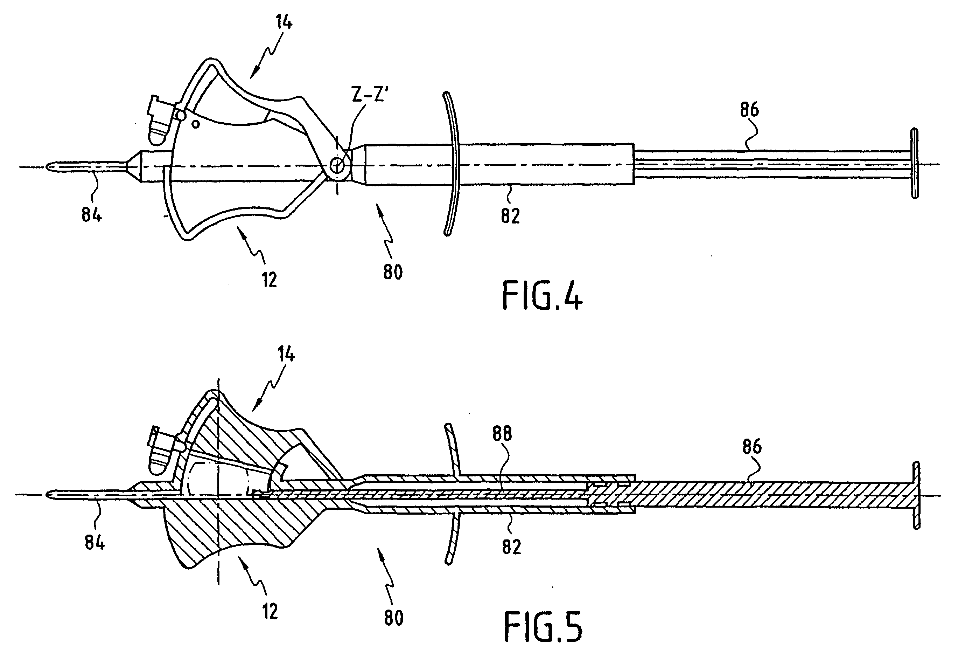

Les figures 4 à 6 illustrent un mode de réalisation d'un injecteur 80 d'implant souple utilisant un module de pliage du type représenté sur les figures 1 à 3. Le premier mors 12 est prolongé à une extrémité par le corps 82 de l'injecteur. La deuxième extrémité du mors 12 est prolongée par une canule 84 qui prolonge elle-même le passage d'injection 36 du mors. Dans ce cas, l'organe de poussage est supprimé et il est remplacé par un piston 86, dont la partie terminale 88 a un diamètre et une longueur convenables pour pousser l'implant plié dans la canule 84.

## Revendications

1. Module de pliage d'un implant intraoculaire souple en vue de son injection dans l'oeil d'un patient, comprennant
deux mors mobiles (12, 14) l'un par rapport à l'autre, chaque mors comprenant une paroi de pliage (18, 26) constituée par une portion de surface cylindrique et une paroi plane (20, 28) solidaire de la paroi de pliage; les deux parois planes étant disposées dans des plans parallèles ;
un axe de rotation (Z-Z') orthogonal aux parois planes pour contrôler le mouvement relatif de rotation d'un mors par rapport à l'autre ;
l'un des mors pouvant occuper par rapport à l'autre une première position dans laquelle les parois planes sont décalées l'une par rapport à l'autre, par quoi l'implant (I) peut être mis en place sur la paroi plane inférieure (20);
une deuxième position dans laquelle les parois planes sont en regard l'une de l'autre, par quoi les parois de pliage et les parois planes définissent un volume (V1) de confinement de l'implant ; et
une troisième position dans laquelle les parois de pliage sont raccordées entre elles pour définir un volume (V2) sensiblement cylindrique formant une chambre de pliage permettant d'obtenir le pliage final de l'implant.

2. Module de pliage selon la revendication 1, **caractérisé en ce que** le premier desdits mors comporte, en outre, deux parois latérales (30, 32) parallèles audit axe de rotation et solidaire des bords de la paroi plane dudit mors, par quoi lorsque les deux mors sont dans leur 2^{ème} position relative, les parois planes, les parois de pliage et les parois latérales définissent un espace de stockage dans lequel l'implant peut être maintenu dans un état non plié.

3. Module de pliage selon la revendication 2, **caractérisé en ce que** la distance (h) entre les deux parois planes est supérieure à l'épaisseur de l'implant.

4. Module de pliage selon la revendication 3, **caractérisé en ce que** ladite distance entre les parois planes est comprise entre 1 et 3 mm.

5. Module de pliage selon l'une quelconque des revendications 2 à 4, **caractérisé en ce que** lesdits mors comprennent en outre des moyens (42, 44) pour les maintenir dans leur deuxième position relative, par quoi l'implant peut être maintenu dans ledit volume de stockage.

6. Module de pliage selon l'une quelconque des revendications 2 à 5, **caractérisé en ce que** lesdits mors comportent en outre des moyens (46) pour les maintenir dans leur troisième position relative, par quoi l'implant peut être maintenu plié dans la chambre de pliage pour permettre l'injection de l'implant dans l'oeil du patient.

7. Module de pliage selon l'une quelconque des revendications 2 à 6, **caractérisé en ce que** ledit premier mors comprend en outre un passage d'injection (36) à l'une des extrémités de ladite chambre de pliage et dont l'axe est sensiblement confondu avec celui du volume cylindrique défini par les parois de pliage lorsque lesdits mors sont dans leur 3^{ème} position relative.

8. Module de pliage selon la revendication 7, **caractérisé en ce que** ledit premier mors comprend en outre un passage de guidage (38) débouchant à la deuxième extrémité de la chambre de pliage et ayant le même axe que celle-ci et un organe de poussage (50) monté coulissant dans le passage de guidage pour pousser l'implant à l'état plié dans ledit passage d'injection.

9. Injecteur d'implants souples **caractérisé en ce qu'**il comprend :
- un module de pliage selon l'une quelconque des revendications 7 et 8 ;
- un corps d'injecteur (82) comprenant un logement pour recevoir ledit module de pliage une canule d'injection (84) pour prolonger ledit passage d'injection lorsque ledit module de pliage est en place dans ledit logement et un conduit axial de guidage pour prolonger ledit passage de guidage (38) lorsque ledit module de pliage est en place dans ledit logement; et
- un piston (86) dont la tige est montée coulissante dans ledit conduit axial de guidage, par quoi l'extrémité de la tige dudit piston peut coopérer avec une extrémité de l'organe de poussage (50) dudit module de pliage.

10. Injecteur d'implants souples **caractérisé en ce qu'**il comprend:
- un module de pliage selon l'une quelconque des revendications 1 à 6 ;
- une canule (84) prolongeant une première extrémité de ladite chambre de confinement pour guider l'implant dans son état plié ;
- un passage de guidage (38) prolongeant une deuxième extrémité de ladite chambre de pliage ; et
- un piston (86) monté coulissant dans ledit passage de guidage pour pousser l'implant à l'état plié de ladite chambre de pliage dans ladite canule.

## Claims

1. A folder module for folding a flexible intraocular implant for injection into the eye of a patient, the module comprising:
- two jaws that are movable (12, 14) relative to each other, each jaw having a folder wall (18, 26) constituted by a portion of a cylindrical surface and a plane wall (20, 28) secured to the folder wall, the two plane walls lying in parallel planes;
- a pivot axis (Z-Z') orthogonal to the plane walls to control relative pivoting movement of one jaw relative to the other;
- one of the jaws being capable of occupying relative to the other jaw a first position in which the plane walls are spaced apart from each other, thereby enabling the implant (I) to be put into place on the bottom plane wall (20);
- a second position in which the plane walls face each other, whereby the folder walls and the plane walls define a confinement volume (V1) for the implant; and
- a third position in which the folder walls meet each other so as to define a substantially cylindrical volume (V2) forming a folder chamber enabling the implant to be finally folded.

2. A folder module according to claim 1, **characterised in that** the first of said jaws further comprises two side walls (30, 32) parallel to said pivot axis and secured to the edges of the plane wall of said jaw, such that when the two jaws are in their second relative position, the plane walls, the folder walls, and the side walls together define a storage space in which the implant can be held in a non-folded state.

3. A folder module according to claim 2, **characterised in that** the distance (4) between the two plane walls is greater than the thickness of the implant.

4. A folder module according to claim 3, **characterised in that** said distance between the plane walls lies in the range 1 mm to 3 mm.

5. A folder module according to any one of claims 2 to 4, **characterised in that** said jaws further comprise means (42, 44) for holding them in their second relative position, whereby the implant can be held in said storage volume.

6. A folder module according to any one of claims 2 to 5, **characterised in that** said jaws further comprise means (46) for holding them in their third relative position, whereby the implant can be held in the folded state in the folder chamber so as to enable the implant to be injected into the eye of a patient.

7. A folder module according to any one of claims 2 to 6, **characterised in that** said first jaw further comprises an injection passage (36) at one of the ends of said folder chamber on an axis that substantially coincides with the axis of the cylindrical volume defined by the folder walls when said jaws are in their third relative position.

8. A folder module according to claim 7, **characterised in that** said first jaw further comprises a guide passage (38) opening out to the second end of the folder chamber and having the same axis as the folder chamber, and a pusher member (50) slidably mounted in the guide passage for pushing the implant in the folded state into said injection passage.

9. A flexible implant injector, **characterised in that** it comprises:
- a folder module according to claim 7 or claim 8;
- an injector body (82) including a housing for receiving said folder module, an injection cannula (84) for extending said injection passage when said folder module is in place in said housing, and an axial guide duct for extending said guide passage (38) when said folder module is in place in said housing; and
- a piston (86) whose rod is slidably mounted in said axial guide duct, whereby the end of the rod of said piston can co-operate with one end of the pusher member (50) of said folder module.

10. A flexible implant injector, **characterised in that** it comprises:
- a folder module according to any one of claims 1 to 6;
- a cannula (84) extending a first end of said confinement chamber to guide the implant in its folded state;
- a guide passage (38) extending a second end of said folder chamber; and
- a piston (86) slidably mounted in said guide passage to push the implant in the folded state from said folder chamber into said cannula.

## Patentansprüche

1. Modul zum Falten eines flexiblen intraokularen Implantats zwecks dessen Injektion in das Auge eines Patienten, umfassend
zwei Backen (12, 14), die in relativ zueinander beweglich sind, wobei jede Backe eine Faltwand (18, 26) umfaßt, die von einem zylindrischen Flächenabschnitt und einer ebenen Wand (20, 28) gebildet wird, die aus einem Stück mit der Faltwand ausgebildet ist, wobei die zwei ebenen Wände in parallelen Ebenen angeordnet sind;
eine Drehachse (Z-Z'), die zu den ebenen Wänden orthogonal verläuft, um die relative Drehbewegung einer Backe zur anderen zu kontrollieren;
wobei die eine der Backen in bezug auf die andere eine erste Position einnehmen kann, in der die ebenen Wände zueinander versetzt sind, wodurch das Implantat (I) auf der unteren ebenen Wand (20) positioniert werden kann;
eine zweite Position, in der die ebenen Wände einander gegenüberliegen, wodurch die Faltwände und die ebenen Wände ein Eingrenzungsvolumen (V1) des Implantats definieren; und
eine dritte Position, in der die Faltwände miteinander verbunden sind, um ein im wesentlichen zylindrisches Volumen (V2) zu definieren, das eine Faltkammer ausbildet, die das Erhalten des endgültigen Faltens des Implantats ermöglicht.

2. Modul zum Falten nach Anspruch 1, **dadurch gekennzeichnet, daß** die erste der Backen ferner zwei seitliche Wände (30, 32) aufweist, die zu der Drehachse parallel verlaufen und mit den Kanten der ebenen Wand der Backe fest verbunden sind, wodurch, wenn sich die zwei Backen in ihrer zweiten relativen Position befinden, die ebenen Wände, die Faltwände und die seitlichen Wände einen Aufbewahrungsraum bilden, in dem das Implantat in einem nicht gefalteten Zustand aufbewahrt werden kann.

3. Modul zum Falten nach Anspruch 2, **dadurch gekennzeichnet, daß** der Abstand (h) zwischen den zwei ebenen Wänden größer ist als die Dicke des Implantats.

4. Modul zum Falten nach Anspruch 3, **dadurch gekennzeichnet, daß** der Abstand zwischen den ebenen Wänden zwischen 1 und 3 mm beträgt.

5. Modul zum Falten nach einem der Ansprüche 2 bis 4, **dadurch gekennzeichnet, daß** die Backen ferner Mittel (42, 44) umfassen, um sie in ihrer zweiten relativen Position zu halten, wodurch das Implantat in dem Aufbewahrungsvolumen aufbewahrt werden kann.

6. Modul zum Falten nach einem der Ansprüche 2 bis 5, **dadurch gekennzeichnet, daß** die Backen des weiteren Mittel (46) umfassen, um sie in ihrer dritten relativen Position zu halten, wodurch das Implantat in der Faltkammer gefaltet aufbewahrt werden kann, um das Injizieren des Implantats in das Auge des Patienten zu gestatten.

7. Modul zum Falten nach einem der Ansprüche 2 bis 6, **dadurch gekennzeichnet, daß** die erste Backe des weiteren einen Injektionsdurchlaß (36) an dem einen der Enden der Faltkammer umfaßt, und deren Achse im wesentlichen mit derjenigen des zylindrischen Volumens zusammenfällt, das durch die Faltwände definiert wird, wenn die Backen sich in ihrer dritten relativen Position befinden.

8. Modul zum Falten nach Anspruch 7, **dadurch gekennzeichnet, daß** die erste Backe des weiteren einen Führungsdurchlaß (38) umfaßt, der an dem zweiten Ende der Faltkammer einmündet und die gleiche Achse wie diese und ein Schiebeorgan (50) aufweist, das gleitend in dem Führungsdurchlaß befestigt ist, um das Implantat in gefaltetem Zustand in den Injektionsdurchlaß zu schieben.

9. Werkzeug zum Einsetzen von flexiblen Implantaten, **dadurch gekennzeichnet, daß** es umfaßt:
- ein Modul zum Falten nach einem der Ansprüche 7 und 8,
- einen Injektionskörper (82), umfassend eine Aufnahme zum Aufnehmen des Moduls zum Falten, eine Injektionskanüle (84) zum Verlängern des Injektionsdurchlasses, wenn sich das Modul zum Falten in der Aufnahme befindet, und eine axiale Führungsleitung zum Verlängern des Führungsdurchlasses (38), wenn sich das Faltmodul in der Aufnahme befindet, und
- einen Kolben (86), dessen Stange gleitend in der axialen Führungsleitung befestigt ist, wodurch das Ende der Stange des Kolbens mit einem Ende des Schiebeorgans (50) des Faltmoduls zusammenwirken kann.

10. Werkzeug zum Einsetzen von flexiblen Implantaten, **dadurch gekennzeichnet, daß** es umfaßt:
- ein Modul zum Falten nach einem der Ansprüche 1 bis 6;
- eine Kanüle (84), die ein erstes Ende der Eingrenzungskammer verlängert, um das Implantat in seinem gefalteten Zustand zu führen;
- einen Führungsdurchlaß (38), der ein zweites Ende der Faltkammer verlängert, und
- einen Kolben (36), der gleitend in dem Führungsdurchlaß befestigt ist, um das Implantat im gefalteten Zustand aus der Faltkammer in die Kanüle zu schieben.
